# FASCICULE DE BREVET EUROPEEN

(11) **EP 0 554 134 B1**
(45) Date de publication et mention de la délivrance du brevet: **22.04.1998**
(21) Numéro de dépôt: 93400087.8
(22) Date de dépôt: 15.01.1993
(51) Int. Cl.: C07C 51/60

(54) **Procédé de synthèse d'halogénures d'acides et réactif utile pour sa mise en oeuvre**
Verfahren zur Herstellung von Carbonsäurehalogeniden und Reaktionsmittel hierzu
Process for preparing carboxylic acid halogenides and a reaction agent therefor

(30) Priorité: 29.01.1992 FR 9200964
(43) Date de publication de la demande: 04.08.1993
(73) Titulaire: RHODIA CHIMIE, 92408 Courbevoie Cédex (FR)
(72) Inventeur: Forat, Gérard, F-69003 Lyon (FR); Gilbert, Laurent, F-69007 Lyon (FR)
(74) Mandataire: Ricalens, François

(56) Documents cités:
- EP-A- 0 166 293
- FR-A- 2 066 768
- GB-A- 926 811
- PATENT ABSTRACTS OF JAPAN vol. 6, no. 165 (C-121)(1043) 28 Août 1982 & JP-A-57 082 336 ( MITSUBISHI GAS KAGAKU K.K. ) 22 Mai 1982

## Description

La présente invention a pour objet un nouveau système catalytique et un procédé de synthèse d'halogénure d'acides, notamment à partir de haloformes. Il concerne plus particulièrement un système catalytique et un procédé permettant de remplacer des fonctions oxyles par des groupes halogénures à partir de haloformes substitués par un reste à caractère nucléophile.

Parmi les nombreuses techniques qui permettent de réaliser la synthèse d'halogénures et plus particulièrement de chlorure d'acides, il convient de citer celles qui consistent à réaliser l'échange entre le phénylchloroforme et l'acide dont on désire former le chlorure. Dans un premier temps on a proposé d'utiliser des acides de Bronstedt puis dans un deuxième temps d'utiliser du chlorure de zinc (cf. la demande de la société HOECHST EPA 0 166 293, page 3 premier paragraphe). Toutefois dans de très nombreux cas la réaction, ou est trop lente, cas des acides de Bronstedt, ou conduit à de nombreuses impuretés, notamment à beaucoup de lourds. Cela est fort gênant car l'on forme d'une part moins du chlorure d'acide désiré et d'autre part moins de chlorure de benzoyle, lequel peut dans certains cas constituer un composé valorisable.

C'est pourquoi, un des buts de la présente invention est de fournir un procédé qui permette de concilier à la fois une grande vitesse de réaction et la production de produits avec un bon rendement. Un autre but de la présente invention est de définir un nouveau système catalytique qui permette une faible production des lourds.

Un autre but de la présente invention est de fournir un procédé qui soit susceptible de réaliser la formation de chlorure d'acides à partir de dérivés réputés difficiles tels que par exemple, la formation de chlorure de trifluoroacétyle à partir d'acide trifluoroacétique.

Ces buts et d'autres qui apparaîtront par la suite sont atteints au moyen d'un procédé d'halogénation au moyen de chloroforme dans lequel l'on emploie comme catalyseur un chlorure de métal, avantageusement trivalent, dont la solubilité dans le mélange réactionnel est au moins égale à une millimole de préférence 1/100 mole par litre, ledit chlorure métallique étant au moins pour moitié avantageusement pour les deux tiers de préférence pour les trois quarts à l'état dissous, le substrat étant de formule R-COOR' où R est choisi parmi formé les radicaux perfluorés R_{f} et ou R' est choisi parmi l'hydrogène les groupes alcoyles ou acyles.

La réaction des chloroformes sur les dérivés des acides carboxyliques usuels, était connue de l'homme de métier, notamment par les documents USP N°926 811, FR A N°2 066 768 et patent abstract of Japan Vol. 6, N°165 (C-121)(1043).

Il est souhaitable qu'il y ait le moins possible dudit chlorure métallique non-dissous et que l'on tombe en deçà des seuils à partir desquels sa présence sous forme hétérogène à la phase réactifère (c'est-à-dire qui contient les réactifs et où a lieu la réaction) est aisément décelée.

Il est à noter que les chlorures d'acides formés au cours de la réaction aident à la dissolution dans le mélange réactionnel et qu'il peut être opportun de maintenir une teneur au moins égale à une fois, avantageusement cinq fois, de préférence dix fois celle dudit chlorure métallique. Cet aspect est particulièrement intéressant lorsque l'on met en oeuvre l'invention en continu.

Le chlorure de métal peut être introduit directement ou sous une forme susceptible de donner dans le milieu réactionnel les mêmes espèces que celles formées par l'addition directe dudit chlorure métallique. Par exemple, on peut introduire le chlorure métallique préféré, à savoir le chlorure ferrique sous la forme de chlorure ferreux dès lors que l'on se trouve dans un milieu oxydant.

Avantageusement, ledit chlorure métallique est le chlorure ferrique.

La concentration en ledit chlorure métallique dissous, à partir de laquelle ce dernier montre une activité significative est de 100 ppm, exprimé en rapport molaire comme détaillé ci-après. Avantageusement la concentration en chlorure métallique est telle que le rapport entre la concentration en chlorure métallique exprimée en moles par litre et la concentration en fonction haloforme, de préférence chloroforme, exprimée en équivalent-gramme par litre soit comprise entre 200 ppm et 5 % de préférence entre 0,1 % et 1 %.

Il est préférable de travailler à des températures relativement modérées. C'est pourquoi, on choisit en général une température comprise entre l'ambiante et 100°C (dans la présente description, les zéros de position ne sont pas des chiffres significatifs, à moins que cela ne soit précisé). Cette limite peut être portée à 200°C lorsque les haloformes sont des fluoroformes ou lorsque de manière plus générale les réactif sont réputé paresseux comme c'est le cas des agents d'halogénation comportant plus de une fonction haloforme sur un même noyau.

Avantageusement, la température est comprise entre 30°C et 100°C, de préférence entre 50°C et 80°C.

La pression ne joue pas un rôle essentiel mais il est possible, afin de pouvoir récupérer un des produits de la réaction au moyen d'une distillation au fur et à mesure de sa formation, de pouvoir travailler à des pressions qui pourront varier entre 10³ et 100.10⁵ de préférence entre 10⁴ et 50.10⁵ Pascals.

Le choix de la pression est déterminé par le choix des substrats et des réactifs ainsi que cela sera spécifié ci-après.

La présente invention est applicable à un vaste domaine de substrats.

Pour former l'halogénure d'acide de formule R-COX, on peut partir notamment d'un substrat de formule R-COO-R'- où R est choisi parmi le groupe constitué par les radicaux alcoyles (de préférence ne portant pas de double liaison conjuguée avec la fonction carbonyle pour éviter les polymérisations intempestives), aryles ou aralcoyles et où R' est choisi parmi l'hydrogène, les groupes alcoyles ou acyles.

Dans la présente description O-R' constitue un groupe oxyle.

Dans ce dernier cas, le substrat forme avantageusement un anhydride externe de préférence symétrique, ou interne.

Parmi les radicaux R, il convient de signaler que l'invention est particulièrement intéressante dans le cas où les alcoyles sont perfluorés (R = R_{f}).

Sont réputés perfluorés les radicaux dont les deux premiers carbones (en α et β du groupe carboxylique ou du groupe qui en dérive) sont perfluorés.

les radicaux alcoyles, aryles ou aralcoyles doivent être pris ici lato sensu.

Il est souhaitable que au moins un des halogénures (en général chlorures) d'acide(s) soit récupéré au fur et à mesure de sa formation. Cette caractéristique est suffisamment favorable pour qu'elle conduise à élire un substrat ou un réactif plutôt qu'un autre et/ou à déterminer les conditions opératoires notamment la pression et l'éventuel solvant.

Aussi lorsque l'on produit des chlorures de perfluoroacyles et notamment de trifluoroacétyle, il est avantageux d'utiliser la faculté des dérivés fortement fluorés d'être très volatils et faire en sorte que le chlorure d'acide formé soit distillé au fur et à mesure de sa formation.

Cette préférence conduit à incliner vers des substrats où R présente une température d'ébullition à pression atmosphérique au plus égale à 180°C.

En ce qui concerne le choix des haloformes, on préfère les chloroformes et les bromoformes bien que la réaction soit également possible pour les fluoroformes à condition de mener la réaction à une température supérieure.

Ainsi, il est souhaitable que l'agent de chloration, à savoir le chloroforme, soit un composé, ou un mélange de composés, de formule Ar-CCl₃ où Ar est un reste présentant un doublet, y compris une insaturation, dans une position où il puisse stabiliser un éventuel carbocation sur l'atome de carbone de la fonction haloforme (en d'autres termes dans le cas où le doublet est une insaturation, les chlores de la fonction chloroforme sont, par exemple et par ordre de préférence, en position propargylique, allylique ou benzylique). Quoique les doublets puissent être amenés par des atomes tels que les halogènes, de préférence le brome et chlore, voire des groupes éthers, on préfère les insaturations, avantageusement de type aryliques. On peut ainsi choisir Ar parmi les groupements ynyles, vinyles et aryles.

Selon la présente invention il est hautement préférable que l'agent halogénant soit toujours présent dans le mélange réactionnel à une teneur au moins égale à une fois avantageusement cinq fois, de préférence dix fois celle dudit chlorure métallique.

Cette condition préférentielle conduit à divers modes de réalisation avantageux.

Ainsi lorsque la production est faite par lots, il est avantageux de ne mener la réaction qu'imparfaitement à son terme. Cela signifie que l'on arrête la réaction dès que ragent halogénant atteint un seuil de transformation de 95% pour les catalyseurs peu efficaces, ou de 99% pour les catalyseurs efficaces, comme le chlorure ferrique.

Que le procédé soit réalisé en continu, en semi-continu ou en discontinu, il est intéressant d'introduire le réactif et le substrat dans le rapport de stoechiométrie; Ainsi avantageusement ledit haloforme est de formule Ar-CX₃ de préférence ArCCl₃ avec X représentant un halogène de préférence un brome un chlore ou un fluor

Le groupement Ar peut porter lui-même d'autres fonctions haloformes qui elles aussi seront réactives. Ainsi, il a été montré au cours de l'étude qui a mené à la présente invention que l'on pouvait de cette façon produire des chlorures d'acides phtaliques à partir des chloroformes correspondants.

A titre indicatif, on peut rappeler que la synthèse de l'ortho di-(trichlorométhyl) benzène est difficile, ce qui réduit l'intérêt économique de cette technique pour produire des dérivés de l'acide ortho-phtalique.

En revanche, cette technique est totalement utilisable pour la synthèse des chlorures d' acides iso- et téréphtalique.

L'analyse des résultats de l'étude qui a mené à la présente invention a montré que les haloformes qui donnaient les réactions les plus propres étaient les composés qui portaient plus de une fonction chloroforme sur le même noyau mais que cela était au détriment de la réactivité. Cette perte de réactivité peut être compensée par une augmentation de la température. un bon compromis doit être trouvé et il est préférable que le nombre de fonction haloforme ne dépasse pas trois sur le même noyau de préférence 2.

La réactivité distincte des diverses fonctions haloformes permet d'obtenir des composés présentant à la fois des fonctions halogénures d'acide et haloformes, fussent les fonctions haloformes identiques. Dans ce cas de figure les calculs relatifs aux conditions obligatoires ou préférentielles selon la présente invention, ne tiendront compte que des fonctions haloformes que l'on désire voire réagir.

Quelqu'intéressante qu'elle soit, cette propriété n'a qu'une importance limitée lorsque les chloroformes sont coûteux ou difficiles à synthétiser.

En général, on préfère utiliser comme solvant les substrats, les agents d'halogènation et les produits issus de la réaction eux-mêmes. Toutefois, lorsque ces réactifs ne sont pas liquides ou ne forment pas un liquide dans les conditions opératoires, il peut être intéressant d'utiliser un solvant, qui pourra également servir pour la régulation de la température, voire de la pression. Les solvants préférés sont choisis parmi le groupe constitué par ceux qui présentent les caractéristiques suivantes :
* inertie relative dans les conditions opératoires ;
* solubilité du dit chlorure métallique au moins égale à une millimole par litre, avantageusement 1/100mole par litre, de préférence 1/10mole par litre.

La réaction peut s'écrire : 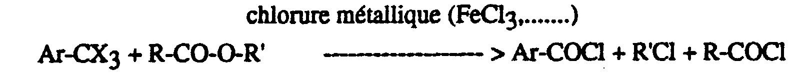 Un des buts supplémentaires de l'invention est de fournir un réactif apte à halogéner les substrats de formule R-COOR' où R est choisi parmi formé les radicaux alcoyles( de préférence ne portant pas de double liaison conjuguées avec la fonction carbonyle pour éviter les polymérisations intempestives), aryles ou aralcoyles et ou R' est choisi parmi l'hydrogène les groupes alcoyles ou acyles.

Ce but est atteint au moyen d'un réactif défini par le fait qu'il contient un chloroforme de formule Ar-CCl₃ où Ar est un reste présentant un doublet, y compris une insaturation, dans une position où il puisse stabiliser un éventuel carbocation sur l'atome de carbone de la fonction haloforme (en d'autres termes dans le cas où le doublet est une insaturation, les chlores de la fonction chloroforme sont, par exemple et par ordre de préférence, en position propargylique, allylique ou benzylique) .

Quoique les doublets puissent être amenés par des atomes tels que les halogènes, de préférence le brome et chlore, voire des groupes éthers, on préfère les insaturations, avantageusement de type aryliques. On peut ainsi choisir Ar parmi les groupements ynyles, vinyles et aryles. Ledit réactif contient un halogénure ferrique, choisi parmi le bromure et le chlorure, à une concentration telle que le rapport entre celle dudit halogénure exprimé en mole par litre et celle des fonctions chloroforme exprimée en équivalent par litre soit compris entre 100 ppm et 5 % de préférence entre 0,1 et 3 %.

Agent d'halogènation et substrats dépassent rarement 20 atomes de carbone et encore plus rarement 50 atomes de carbone.

Les aryles sont avantageusement homocycliques (par opposition à hétérocyclique) et ne comportent pas plus de quatre noyaux, condensés ou non.

Les exemples qui suivent sont non limitatifs et illustrent l'invention.

### EXEMPLES

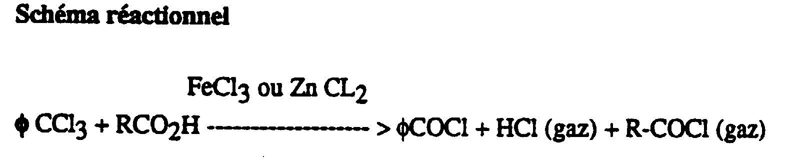

### Exemple 1

Dans un réacteur de 250 ml, on charge 107 g (0,54 mole) de phénylchloroforme et 0,57 g de chlorure ferrique sous atmosphère d'azote, à 0°C. On charge alors 62,5 g (0,548 mole) d'acide trifluoroacétique puis on ferme le réacteur sur une solution homogène. On chauffe lentement de manière à atteindre une température de 60°C après 30 minutes de chauffage. La pression dans le réacteur est maintenue entre 7 et 8.10⁵ Pascal par élimination en continue de la phase gazeuse. La réaction est poursuivie pendant 130 minutes, l'émission de gaz cesse alors. Les produits volatils sont récupérés par dégazage, on refroidit alors l'ensemble et on purge l'installation au moyen d'azote. Les produits non volatils sont récupérés par distillation.

| | |
|---|---|
| La conversion du phénylchloroforme est de | 100 %. |
| Le rendement en chlorure de trifluoroacétyle est de | 98 %. |
| Le rendement en chlorure de benzoyle est de | 93 %. |

Les produits lourds sont obtenus à l'état de traces.

### Exemple comparatif

Un essai semblable est réalisé en utilisant comme catalyseur le chlorure de zinc (1,6g). La solubilité du chlorure de zinc est inférieure à 2000 ppm en poids. La réaction dure cinq heures et les résultats suivants sont obtenus :

| | |
|---|---|
| La conversion du phénylchloroforme est de | 81 %. |
| Le rendement en chlorure de trifluoroacétyle est de | 70 %. |
| Le rendement en chlorure de benzoyle est de | 78 %. |

2 % de lourds ont été mis en évidence.

### Exemple 2

La réaction est réalisée dans un réacteur de 30 ml, à pression atmosphérique. Un mélange équimolaire d'acide trifluoroacétique de phénylchloroforme est additionné en continu à une solution de chlorure ferrique dans le chlorure de benzoyle à 65°C [FeCl₃/(ΦCCl₃total engagé) = 0,15 % molaire] en 4h30.

Les produits volatils formés : chlorure de trifluoroacétyle et chlorure d'hydrogène sont piégés et dosés en continu.

On obtient:

| | |
|---|---|
| Conversion du phénylchloroforme | 100 % |
| Rendement en chlorure de trifluoroécétyle | 98,4 % |
| Rendement en chlorure de benzoyle | 96,6 % |

### Exemple 3

La réaction est réalisée comme dans l'exemple 2 en additionnant à une solution de chlorure ferrique dans l'acide trifluoroacétique à 65°C [FeCl₃/(ΦCCl₃total engagé) = 1,2 % molaire] en 5h un équivalent molaire de phénylchloroforme.

On obtient :

| | |
|---|---|
| Conversion du phénylchloroforme | 100% |
| Rendement en chlorure de trifluoroacétyle | 94,4 % |
| Rendement en chlorure de benzoyle | 92,8 % |

### Exemple 4

La réaction est réalisée comme dans l'exemple 2 en additionnant à une solution de chlorure ferrique dans le phénylchloroforme à 65°C. (FeCl₃/ΦCCl₃ = 1,5 % molaire) en 4h10 un équivalent molaire d'acide trifluoroacétique

On obtient :

| | |
|---|---|
| Conversion du phénylchloroforme | 100 % |
| Rendement en chlorure de trifluoroacétyle | 93,7 % |
| Rendement en chlorure de benzoyle | 81,6 % |

## Revendications

1. Procédé de chloruration au moyen de chloroforme caractérisé par le fait que l'on emploie comme catalyseur un chlorure de métal, avantageusement trivalent, dont la solubilité dans le mélange réactionnel est au moins égale à une millimole de préférence 1/100 mole par litre, ledit chlorure métallique étant au moins pour moitié avantageusement pour les deux tiers de préférence pour les trois quarts à l'état dissous, et par le fait que le substrat est de formule R-COOR' où R est choisi parmi formé les radicaux perfluorés R_{f} et ou R' est choisi parmi l'hydrogène les groupes alcoyles ou acyles.

2. Procédé d'halogénation au moyen de chloroforme selon la revendication 1, caractérisé par le fait que ledit chlorure métallique est le chlorure ferrique.

3. Procédé d'halogénation au moyen de chloroforme selon les revendications 1 et 2, caractérisé par le fait que la concentration en chlorure métallique est telle que le rapport entre la concentration en chlorure métallique exprimée en moles par litre et la concentration en fonction, exprimée en équivalent par litre soit comprise entre 200 ppm et 5 % de préférence entre 0,1 % et 1 %.

4. Procédé d'halogénation au moyen de chloroforme selon les revendications 1 à 3, caractérisé par le fait que ledit chloroforme est de formule ArCCl₃.

5. Procédé d'halogénation au moyen de chloroforme selon l'une des revendications 1 à 4, caractérisé par le fait que la température est comprise entre 30°C et 100°C, de préférence entre 50°C et 80°C

6. Procédé d'halogénation au moyen selon l'une des revendications 1 à 5, caractérisé par le fait que Ar est un reste présentant un doublet, y compris une insaturation, dans une position où il puisse stabiliser un éventuel carbocation sur l'atome de carbone de la fonction chloroforme

7. Procédé d'halogénation au moyen de chloroforme selon l'une des revendications 1 à 6, caractérisé par le fait Ar est choisi que parmi les groupements ynyles, vinyles et aryles..

8. Procédé d'halogénation au moyen de chloroforme selon l'une des revendications 1 à 7, caractérisé par le fait que ledit chloroforme est présent dans le mélange réactionnel à une teneur au moins égale à une fois avantageusement cinq fois, de préférence dix fois celle dudit chlorure métallique.

## Patentansprüche

1. Verfahren zur Chlorierung mit Chloroform, dadurch **gekennzeichnet**, daß man als Katalysator ein bevorzugt dreiwertiges Metallchlorid verwendet, dessen Löslichkeit in dem Reaktionsgemisch mindestens 1 mmol, bevorzugt 1/100 mol pro 1, beträgt, wobei das Metallchlorid mindestens zur Hälfte, bevorzugt zu zwei Drittel, bevorzugt zu drei Viertel, in gelöstem Zustand vorliegt, und daß das Substrat die Formel R-COOR' besitzt, worin R aus perfluorierten Resten R_{f} ausgewählt ist und/oder R' aus Wasserstoff, Alkyl- oder Acylresten ausgewählt ist.

2. Verfahren zur Halogenierung mit Chloroform nach Anspruch 1, dadurch **gekennzeichnet,** daß das Metallchlorid Eisen(III)-chlorid ist.

3. Verfahren zur Halogenierung mit Chloroform nach den Ansprüchen 1 und 2, dadurch **gekennzeichnet,** daß die Konzentration des Metallchlorids so ist, daß das Verhältnis zwischen der Konzentration des Metallchlorids, ausgedrückt als mol pro 1, und der Konzentration der Funktion, ausgedrückt als Äquivalent pro 1, zwischen 200 ppm und 5 %, bevorzugt zwischen 0,1 % und 1 %, liegt.

4. Verfahren zur Halogenierung mit Chloroform nach den Ansprüchen 1 bis 3, dadurch **gekennzeichnet,** daß das Chloroform die Formel ArCCl₃ besitzt.

5. Verfahren zur Halogenierung mit Chloroform nach einem der Ansprüche 1 bis 4, dadurch **gekennzeichnet,** daß die Temperatur zwischen 30°C und 100°C, bevorzugt zwischen 50°C und 80°C, liegt.

6. Verfahren zur Halogenierung mit Chloroform nach einem der Ansprüche 1 bis 5, dadurch **gekennzeichnet,** daß Ar ein Rest mit einem Dublett einschließlich einer Unsättigung in einer Position ist, in der diese ein evtl. vorhandenes Carbokation am Kohlenstoffatom der Chloroformfunktion stabilisieren kann.

7. Verfahren zur Halogenierung mit Chloroform nach einem der Ansprüche 1 bis 6, dadurch **gekennzeichnet,** daß Ar aus den Resten Inyl, Vinyl und Aryl ausgewählt wird.

8. Verfahren zur Halogenierung mit Chloroform nach einem der Ansprüche 1 bis 7, dadurch **gekennzeichnet,** daß das Chloroform in dem Reaktionsgemisch in einem Gehalt von mindestens dem einfachen, vorteilhafterweise dem fünffachen, bevorzugt dem zehnfachen der Konzentration des Metallchlorids vorhanden ist.

## Claims

1. Process for chlorinating by means of chloroform, characterized in that a metal chloride, advantageously trivalent, is used as catalyst, the solubility of which in the reaction mixture is at least equal to one millimole, preferably 1/100 mol, per litre, the said metal chloride being at least half, advantageously two-thirds and preferably three-quarters in the dissolved state, and in that the substrate is of formula R-COOR¹, where R is chosen from perfluorinated radicals R_{F} and where R¹ is chosen from hydrogen or alkyl or acyl groups.

2. Process for halogenating by means of chloroform according to claim 1, characterized in that the said metal chloride is ferric chloride.

3. Process for halogenating by means of chloroform according to claims 1 and 2, characterized in that the concentration of metal chloride is such that the ratio of the concentration of metal chloride, expressed in moles per litre, to the concentration of functional group, expressed in equivalents per litre, is between 200 ppm and 5%, preferably between 0.1% and 1%.

4. Process for halogenating by means of chloroform according to claims 1 to 3, characterized in that the said chloroform is of formula ArCCl₃.

5. Process for halogenating by means of chloroform according to one of claims 1 to 4, characterized in that the temperature is between 30°C and 100°C, preferably between 50°C and 80°C.

6. Process for halogenating by means of chloroform according to one of claims 1 to 5, characterized in that Ar is a residue having a doublet, including an unsaturation, in a position where it can stabilise a possible carbocation on the carbon atom of the chloroform functional group.

7. Process for halogenating by means of chloroform according to one of claims 1 to 6, characterized in that Ar is chosen from the ynyl, vinyl and aryl groups.

8. Process for halogenating by means of chloroform according to one of claims 1 to 7, characterized in that the said chloroform is present in the reaction mixture at a content at least equal to one times, advantageously five times, preferably ten times that of the said metal chloride.
